Europäisches Patentamt

European Patent Office

Office européen des brevets

(11) Veröffentlichungsnummer: **0 107 823**
**A2**

(12) # EUROPÄISCHE PATENTANMELDUNG

(21) Anmeldenummer: 83110003.7

(22) Anmeldetag: 06.10.83

(51) Int. Cl.³: **C 12 N 15/00**
**C 12 N 9/84, C 07 H 21/04**

(30) Priorität: 21.10.82 DE 3238976
19.08.83 DE 3330003

(43) Veröffentlichungstag der Anmeldung:
09.05.84 Patentblatt 84/19

(84) Benannte Vertragsstaaten:
AT BE CH DE FR GB IT LI NL SE

(71) Anmelder: Gesellschaft für Biotechnologische
Forschung mbH (GBF)
Mascheroder Weg 1
D-3300 Braunschweig-Stöckheim(DE)

(72) Erfinder: Brüning, Heinrich, Dr. Dipl.-Bio.
Solenander Strasse 2
D-4000 Düsseldorf(DE)

(72) Erfinder: Bruns, Wolfgang, Dr. Dipl.-Chem.
Hahnenklee 7
D-3300 Braunschweig(DE)

(72) Erfinder: Collins, John, Dr.
Salzdahlumer Weg 5
D-3300 Braunschweig(DE)

(72) Erfinder: Hahn, Wilfried
Rüninger Weg 50
D-3300 Braunschweig(DE)

(72) Erfinder: Hoppe, Jürgen, Dr. Dipl.-Chem.
Zickerickstrasse 36
D-3340 Wolfenbüttel(DE)

(72) Erfinder: Mayer, Hubert, Dr.
Breite Herzogstrasse 24
D-3340 Wolfenbüttel(DE)

(74) Vertreter: Boeters, Hans Dietrich, Dr. et al,
Boeters, Bauer & Partner Thomas-Wimmer-Ring 14
D-8000 München 22(DE)

(54) DNA-Sequenz und DNA-Strukturen und sie verwendendes Verfahren zur Herstellung von Penicillin-acylase.

(57) Die Erfindung betrifft DNA-Sequenzen und DNA-Strukturen und ein sie verwendendes Verfahren.

EP 0 107 823 A2

Croydon Printing Company Ltd

Anmelderin: Gesellschaft für Biotechnologische
Forschung mbH (GBF), 3300 Braunschweig-Stöckheim

DNA-Sequenz und DNA-Strukturen und sie verwendendes Verfahren zur Herstellung von Penicillin-acylase.

Es ist bekannt, daß diverse Mikroorganismen Penicillin-amidase EC 3.5.1.11 bzw. Penicillin-acylase produzieren und daß
das Penicillin-acylasegen z.B. aus E. coli ATCC 11105 kloniert ist und als 5K (pHM12) = DSM 1610 = NCIB 11670 vorliegt
(DE-OS 29 30 794.6). Nach Rothstein et al, Cold Spring Harbor
Symposia on quantitative Biology, Vol. XLV (1981) 99 – 105,
ist die Lokalisierbarkeit eines Gens durch Insertions-Inaktivierung eines Transposons bekannt. Die DNA-Sequenzierung ist
nach Maxam & Gilbert, Methods Enzymology 65 (1980) 499 – 560,
bekannt. Die Proteinsequenzierung ist nach Laursen, Methods
Enzymology 25 (1972) 344 – 359, bekannt. Das gezielte Ankoppeln eines Gens an einen starken gegebenenfalls induzierbaren
Promotor ist nach Pribnow, Biological Regulation and Development, Bd. 1, 231–277, Plenum Press No. 4 (Goldberger et al.
1979), bekannt.

Es ist nun Aufgabe der Erfindung, das Gen für die Penicillin-acylase auf einem der Klone zu lokalisieren und damit
die Voraussetzung für gezielte Stammverbesserung zu schaffen.

Gegenstand der Erfindung ist einerseits eine DNA-Sequenz, die
durch folgende Merkmale gekennzeichnet ist:

(a) die DNA-Sequenz kodiert eine der Komponenten der Penicillin-acylase, die - aus einer ca. 90k-Vorform - im reifen
Zustand insbesondere in E.coli ein 2-Komponenten-Aggregat
darstellt, in Form einer ca. 20k-Untereinheit (ca. 20 kD)
der folgenden aminoterminalen Proteinsequenz:

$H_2N$-Glu-Gln-Ser-Ser-Ser-Glu-Ile-Lys-Ile-Val-Arg-Asp- usw.
    GAG CAG TCG TCA AGT GAG ATA AAG ATT GTT CGC GAT  usw.

oder die DNA-Sequenz kodiert nur einen Teil dieser einen
Komponente der Penicillin-acylase,
(b) wobei die DNA-Sequenz gemäß (a) gegebenenfalls hinter
einen Promotor oder einen Promotor/Leader geschaltet sein
kann,
(c) wobei die DNA-Sequenz gewinnbar ist aus ATCC 11105,

sowie zu (a) analoge DNA-Sequenzen, die aus Mikroorganismen,
insbesondere E.-coli-Stämmen gewinnbar sind, die Penicillin-acylase produzieren, wobei diese analogen DNA-Sequenzen
gegebenenfalls das Merkmal (b) aufweisen.

Gegenstand der Erfindung ist ferner eine DNA-Sequenz, die
durch folgende Merkmale gekennzeichnet ist:

(d) die DNA-Sequenz kodiert die andere Komponente der Penicillin-acylase in Form einer ca. 66k-Untereinheit der folgenden aminoterminalen Proteinsequenz:

H$_2$N-Ser-Asn-Met-Trp-Val-Ile-Gly-Lys-Ser-Lys-Ala-Gln-Asp- usw.
AGC AAT ATG TGG GTG ATC GGC AAA AGC AAA GCC CAG CAT usw.


oder die DNA-Sequenz kodiert nur einen Teil dieser anderen
Komponente der Penicillin-acylase,
(e) wobei der DNA-Sequenz gemäß (d) gegebenenfalls ein Promotor oder ein Promotor/Leader vorgeschaltet sein kann,
(f) wobei die DNA-Sequenz gewinnbar ist aus ATCC 11105,


sowie zu (d) analoge DNA-Sequenzen, die aus Mikroorganismen,
insbesondere E.-coli-Stämmen gewinnbar sind, die Penicillin-acylase produzieren, wobei diese analogen DNA-Sequenzen
gegebenenfalls das Merkmal (e) aufweisen.


Gegenstand der Erfindung ist ferner eine DNA-Sequenz (DNA-Gesamtsequenz), die durch folgende Merkmale gekennzeichnet ist:


(g) eine DNA-Sequenz (DNA-Untersequenz) gemäß (a),
(h) eine DNA-Sequenz (DNA-Untersequenz) gemäß (d), die (gelesen ab AGC) auf die DNA-Sequenz gemäß (a) folgt;
(i) daß die DNA-Sequenz (DNA-Untersequenz) gemäß (a) gegebenenfalls hinter einen Promotor oder Promotor/Leader gemäß
(b) geschaltet sein kann, der für beide DNA-Untersequenzen
verantwortlich ist,


sowie DNA-Sequenzen (DNA-Gesamtsequenzen), die zu der DNA-Sequenz mit den Merkmalen (g), (h) und ggf. (i) analog und aus
Mikroorganismen gewinnbar sind (beispielsweise Bacillus), die
Penicillin-acylase produzieren,


sowie DNA-Sequenzen (DNA-Gesamtsequenzen), die zu (g) und (h)
analoge DNA-Untersequenzen umfassen und aus Mikroorganismen,
insbesondere E.-coli-Stämmen gewinnbar sind, die Penicillin-
acylase produzieren, wobei diese analoge DNA-Untersequenzen

- 4 -

umfassenden DNA-Sequenzen gegebenenfalls das Merkmal (i)
aufweisen.

Die DNA-Sequenz (DNA-Gesamtsequenz) mit den Merkmalen (g),
(h) und (i) kann ca. $2,6 \times 10^3$ Basenpaare (2,6 kb) z.B. bei
E. coli umfassen.

Für Promotoren vgl. man beispielsweise Pribnow, loc. cit. Für
Leader und Promotor/Leader-Kombinationen vgl. man beispielsweise Inouye & Halegoua, Crit. Rev. Biochem., (1980) 339-371.
Leader sorgen dafür, daß exprimiertes Protein aus dem Zytoplasmabereich in das Periplasma oder aus der Zelle wandern
kann.

Aus ATCC 11105 lassen sich beispielsweise ein Promotor und
eine Promotor/Leader-Kombination gemäß Schema 1 isolieren:

- der Promotor endet mit ACA (Thr) und reicht ca. 300 Basenpaare nach links;
- der Leader beginnt mit ATG (Met) und endet mit GCT (Ala).

Gegenstand der Erfindung sind ferner klonierbare DNA-Strukturen (Vektoren), in denen die genannten erfindungsgemäßen
DNA-Sequenzen enthalten sind.

Die Erfindung sieht ferner ein Verfahren zur Herstellung von
Penicillin-acylase vor, das dadurch gekennzeichnet ist, daß
man

- von einer DNA-Sequenz mit den Merkmalen (g) und (h) und
gewinnbar aus ATCC 11 105 den Promotor oder Promotor/Leader
gemäß Schema 1 gegen einen anderen Promotor oder Promotor/
Leader austauscht und

- mit Hilfe des Austauschprodukts einen prokaryotischen
Mikroorganismus Penicillin-acylase bilden läßt.

Anstelle der aus ATCC 11 105 gewinnbaren DNA-Sequenz mit den
.Merkmalen (g), (h) und (i) kann man in das erfindungsgemäße
Verfahren dazu analoge DNA-Sequenzen einsetzen, die aus
Mikroorganismen, insbesondere E.-coli-Stämmen gewonnen worden
sind, die Penicillin-acylase produzieren, wobei man die
Promotoren oder Promotoren/Leader der gewonnenen DNA-Sequenzen gegen andere Promotoren oder Promotoren/Leader austauscht.

Die Erfindung sieht ferner ein Verfahren zur Herstellung von
Penicillin-acylase vor, das dadurch gekennzeichnet ist, daß
man

- die DNA-Sequenz gemäß (a), die nur die ca. 20k-Untereinheit
kodiert, hinter einen ggf. induzierbaren Promotor oder Promotor/Leader schaltet,
- die DNA-Sequenz gemäß (d), die nur die ca. 66k-Untereinheit
kodiert, hinter einen ggf. induzierbaren Promotor oder Promotor/Leader schaltet und

- die beiden Untereinheiten von einem prokaryotischen Mikroorganismus bilden läßt (wobei jedes Individuum des Mikroorganismus beide Untereinheiten bildet) und gegebenenfalls
extrahiert oder
- die beiden Untereinheiten für sich getrennt in verschiedenen Individuen eines prokaryotischen Mikroorganismus bilden
läßt, gegebenenfalls extrahiert und gegebenenfalls danach
zusammengibt und
- gegebenenfalls danach die gebildete Penicillin-acylase
isoliert.

Indem man die DNA-Sequenz gemäß (a) und gemäß (d) jeweils
hinter einen Promotor schaltet, lassen sich erhöhte Ausbeuten
an Penicillin-acylase erreichen.

Anstelle der DNA-Sequenzen gemäß (a) und (d) kann man in das
erfindungsgemäße Verfahren dazu analoge DNA-Sequenzen einsetzen, die aus Mikroorganismen, insbesondere E.-coli-Stämmen gewonnen worden sind, die Penicillin-acylase produzieren.

Als Promotor oder Promotor/Leader kann man in das erfindungsgemäße Verfahren die aus ATCC 11105 gewonnenen DNA-Sequenzen gemäß Schema 1 einsetzen.

Der Fachmann ist mit prokaryotischen Mikroorganismen (beispielsweise Bacillus und E. coli) vertraut, die sich für das erfindungsgemäße Verfahren verwenden lassen.

Es ist vorteilhaft, beim erfindungsgemäßen Verfahren als prokaryotischen Mikroorganismus (Wirtsstamm) einen Stamm zu verwenden, der eine Protease-Aktivität aufweist, die der von E. coli 5K entspricht oder größer ist. Ein Beispiel für einen E.-coli-5K-Stamm ist DSM 1454. Für protease-aktive E.-coli-Stämme vgl. auch Date & Wickner, Proc. Natl. Acad. Sci. USA 78 (1981) 6106 - 6110. Durch derartige Stämme wird der Leader besser abgespalten und gegebenenfalls das aus zwei Komponenten bestehende Protein besser zerlegt.

Die Erfindung wird durch die nachstehenden Beispiele näher erläutert.

Beispiel 1

Die Penicillinacylase wurde mit Hilfe von 5K (pHM12) als Aggregat isoliert. Die Untereinheiten 20k und 66k wurden durch HPLC-Chromatographie getrennt und ferner wurden Aminosäuren vom $NH_2$-Terminus beider Untereinheiten bestimmt.
20k hat folgende Aminosäuren: $H_2N$-Glu-Gln-Ser-Ser-Ser-Glu-Ile-Lys-Ile-Val-Arg-Asp-. 66k hat folgende Aminosäuren: $H_2N$-Ser-Asn-Met-Trp-Val-Ile-Gly-Lys-Ser-Lys-Ala-Gln-Asp-.

## Beispiel 2

Das EcoRI-HindIII-Fragment wurde von pHM12 isoliert und die
DNA-Sequenz ermittelt. Von der HindIII-Schnittstelle gelesen
in Richtung auf die EcoRI-Schnittstelle lautet sie folgendermaßen:

A AGC TTC GTT GCT AGT ATC AAT TCG CTA ATT ATA CAC CTG CCA GAG

GAT ACA*ATG AAA AAT AGA AAT CGT ATG ATC GTG AAC TGT GTT ACT

       -Met-Lys-Asn-Arg-Asn-Arg-Met-Ile-Val-Asn-Cys-Val-Thr-

GCT TCC CTG ATG TAT TAT TGG AGC TTA CCT GCA CTG GCT*GAG CAG

Ala-Ser-Leu-Met-Tyr-Tyr-Trp-Ser-Leu-Pro-Ala-Leu-Ala-Glu-Gln

*ATG: Promotor-Ende und Leader-Beginn
*GAG: Leader-Ende und Beginn der ca. 20k-Untereinheit

Sie kodiert für den Beginn der 20k-Untereinheit. Vorausgehende Tripletts kodieren für eine Vorläufersequenz, die im
reifen Protein nicht auftritt. Damit ist der Beginn des
Gens für Penicillinacylase exakt bestimmt, so daß die Voraussetzung geschaffen worden ist, das Gen gezielt hinter
einen starken und gegebenenfalls induzierbaren Promotor
zu schalten, um Mikroorganismen mit erhöhter Acylaseproduktivität zu konstruieren.

## Beispiel 3

Aus pHM12 wird das vollständige Penicillin-acylase-Gen isoliert. Das Gen wird gemäß Beispiel 1 der DE-PS 28 14 039.8
in E.-coli überführt und kloniert. Die DNA wird mit HindIII
geschnitten. Danach wird die DNA-Sequenz, die die 66 kD-Untereinheit kodiert, hinter die Promotor/Leader-Sequenz gemäß
Schema 1 geschaltet. Die beiden DNA-Sequenzen, die die beiden
Enzym-Untereinheiten kodieren, werden getrennt gemäß Beispiel 1 der DE-PS 28 14 039.8 in E. coli überführt und

kloniert. Die separat gebildeten Enzym-Untereinheiten werden extrahiert und danach zusammengegeben. Die Ausbeute an Penicillin-acylase beträgt in einem 1 l-Reaktor 0,1 g.

Beispiel 4

DNA wird aus Bacillus megaterium ATCC 14 945 gewonnen und mit HpaI, EcoRI + PstI, NcoI geschnitten, auf einem Agarosegel aufgetrennt, auf Nitrocellulosepapier transferiert und mit radioaktiv markiertem Penicillin-acylasegen aus pHM12 hybridisiert. Zum Penicillin-acylasegen homologe DNA-Regionen sind auf dem Röntgenfilm als definierte Banden sichtbar. Das Penicillin-acylasegen aus Bacillus megaterium ist also dem Penicillin-acylasegen aus pHM12 analog.

Schema 1

T C T T T T A G A T C A C A T T A A T G

A A A T T T T T G T A T C A A A A A T T A G T T A T C G C G

C T C A C A G T T C A T A A T G A A A C

A A T T C T C T G C A A A T A G A T A A A C C G A A G C T T C

G T T G C T A G T A T C A A T T C G C T A A T T A T A C A C

```
    -Leu-  -Pro-  -Glu-  -Asp-  -Thr-  -Met-  -Lys-  -Asn-  -Arg-  -Asn-
   C T G C C A G A G G A T A C A A T G A A A A A T A G A A A T
              Promotorende ↑ Leaderbeginn

    -Arg-  -Met-  -Ile-  -Val-  -Asn-  -Cys-  -Val-  -Thr-  -Ala-  -Ser-
   C G T A T G A T C G T G A A C T G T G T T A C T G C T T C C

    -Leu-  -Met-  -Tyr-  -Tyr-  -Trp-  -Ser-  -Leu-  -Pro-  -Ala-  -Leu-  -Ala-
   C T G A T G T A T T A T T G G A G C T T A C C T G C A C T G G C T
```

Patentansprüche

1. DNA-Sequenz, g e k e n n z e i c h n e t durch folgende
Merkmale:

(a) die DNA-Sequenz kodiert eine der Komponenten der Penicillin-acylase in Form einer ca. 20 kD-Untereinheit der
folgenden aminoterminalen Proteinsequenz:

$H_2N$-Glu-Gln-Ser-Ser-Ser-Glu-Ile-Lys-Ile-Val-Arg-Asp- usw.
    GAG CAG TCG TCA AGT GAG ATA AAG ATT GTT CGC GAT usw.,

oder die DNA-Sequenz kodiert nur einen Teil dieser einen
Komponente der Penicillin-acylase,

(b) wobei die DNA-Sequenz gemäß (a) gegebenenfalls hinter
einen Promotor oder einen Promotor/Leader geschaltet sein
kann,

(c) wobei die DNA-Sequenz gewinnbar ist aus ATCC 11105,

sowie zu (a) analoge DNA-Sequenzen, die aus Mikroorganismen,
insbesondere E.-coli-Stämmen gewinnbar sind, die Penicillin-acylase produzieren, wobei diese analogen DNA-Sequenzen
gegebenenfalls das Merkmal (b) aufweisen.

2. DNA-Sequenz, g e k e n n z e i c h n e t  durch folgende
Merkmale:

(d) die DNA-Sequenz kodiert die andere Komponente der Penicillin-acylase in Form einer ca. 66 kD-Untereinheit der
folgenden aminoterminalen Proteinsequenz:

$H_2$N-Ser-Asn-Met-Trp-Val-Ile-Gly-Lys-Ser-Lys-Ala-Gln-Asp- usw.
     AGC AAT ATG TGG GTG ATC GGC AAA AGC AAA GCC CAG CAT usw.

oder die DNA-Sequenz kodiert nur einen Teil dieser anderen
Komponente der Penicillin-acylase,

(e) wobei der DNA-Sequenz gemäß (d) gegebenenfalls ein
Promotor oder ein Promotor/Leader vorgeschaltet sein kann,

(f) wobei die DNA-Sequenz gewinnbar ist aus ATCC 11105,

sowie zu (d) analoge DNA-Sequenzen, die aus Mikroorganismen, insbesondere E.-coli-Stämmen gewinnbar sind, die Penicillin-acylase produzieren, wobei diese analogen DNA-Sequenzen gegebenenfalls das Merkmal (e) aufweisen.

3. DNA-Sequenz, g e k e n n z e i c h n e t  durch folgende
Merkmale:

(g) eine DNA-Sequenz (DNA-Untersequenz) gemäß Anspruch 1
(a),

(h) eine DNA-Sequenz (DNA-Untersequenz) gemäß Anspruch 2
(d), die (gelesen ab AGC) auf die DNA-Sequenz gemäß Anspruch 1 (a) folgt;

(i) daß die DNA-Sequenz (DNA-Untersequenz) gemäß Anspruch 1
(a) gegebenenfalls hinter einen Promotor oder Promotor/Leader gemäß Anspruch 1 (b) geschaltet sein kann, der für
beide DNA-Untersequenzen verantwortlich ist,

sowie DNA-Sequenzen, die zu (g) und (h) analoge DNA-Untersequenzen umfassen und aus Mikroorganismen, insbesondere E.-coli-Stämmen gewinnbar sind, die Penicillin-acylase produzieren, wobei diese analoge DNA-Untersequenzen umfassenden DNA-Sequenzen gegebenenfalls das Merkmal (i) aufweisen.

4. Klonierbare DNA-Strukturen (Vektoren), dadurch  g e - k e n n z e i c h n e t , daß sie eine DNA-Sequenz gemäß Anspruch 1, 2 oder 3 enthalten.

5. Verfahren zur Herstellung von Penicillin-acylase, dadurch  g e k e n n z e i c h n e t , daß man
- von einer DNA-Sequenz gemäß Anspruch 3 mit den Merkmalen (g) und (h) und gewinnbar aus ATCC 11 105 den Promotor oder Promotor/Leader gemäß dem folgenden Schema

TCTTTTAGATCACATTAATG

AAATTTTTGTATCAAAAATTAGTTATCGCG

CTCACAGTTCATAATGAAAC

AATTCTCTGCAAATAGATAAACCGAAGCTTC

!G T T G C T A G T A!T C A A T T C G C T!A A T T A T A C A C!


-Leu- -Pro- -Glu- -Asp- -Thr- -Met- -Lys- -Asn- -Arg- -Asn-
-!C T G C C A G A G G!A T A C A!A T G A A!A A A T A G A A A T!

Promotorende ↑ Leaderbeginn


-Arg- -Met- -Ile- -Val- -Asn- -Cys- -Val- -Thr- -Ala- -Ser-
C G T A T G A T C G!T G A A C T G T G T!A C T G C T T C C!


-Leu- -Met- -Tyr- -Tyr- -Trp- -Ser- -Leu- -Pro- -Ala- -Leu- -Ala-
-!C T G A T G T A T T!A T T G G A G C T T!A C C T G C A C T G!G C T


gegen einen anderen Promotor oder Promotor/Leader austauscht
und
- mit Hilfe des Austauschprodukts einen prokaryotischen
Mikroorganismus Penicillin-acylase bilden läßt.


6. Verfahren zur Herstellung von Penicillin-acylase, dadurch g e k e n n z e i c h n e t ,
daß man in das Verfahren DNA-Sequenzen einsetzt, die aus
Mikroorganismen, insbesondere E.-coli-Stämmen gewonnen
worden sind, die Penicillin-acylase produzieren, und der
DNA-Sequenz gemäß Anspruch 5 mit den Merkmalen (g), (h) und
(i) analog sind,
- wobei man die Promotoren oder Promotoren/Leader der gewonnenen DNA-Sequenzen gegen andere Promotoren oder Promotoren/Leader austauscht und
- mit Hilfe der Austauschprodukte einen prokaryotischen
Mikroorganismus Penicillin-acylase bilden läßt.

7. Verfahren zur Herstellung von Penicillin-acylase, dadurch  g e k e n n z e i c h n e t , daß man
- die DNA-Sequenz gemäß Anspruch 1 (a), die nur die ca. 20 kD-Untereinheit kodiert, hinter einen gegebenenfalls induzierbaren Promotor oder Promotor/Leader schaltet,
- die DNA-Sequenz gemäß Anspruch 2 (d), die nur die ca. 66 kD-Untereinheit kodiert, hinter einen gegebenenfalls induzierbaren Promotor oder Promotor/Leader schaltet und
- die beiden Untereinheiten von einem prokaryotischen Mikroorganismus bilden läßt (wobei jedes Individuum des Mikroorganismus beide Untereinheiten bildet) und gegebenenfalls extrahiert oder
- die beiden Untereinheiten für sich getrennt in verschiedenen Individuen eines prokaryotischen Mikroorganismus bilden läßt, gegegebenenfalls extrahiert und gegebenenfalls danach zusammengibt und
- gegebenenfalls danach die gebildete Penicillin-acylase isoliert.

8. Verfahren zur Herstellung von Penicillin-acylase, dadurch  g e k e n n z e i c h n e t , daß man
- eine DNA-Sequenz, die aus einem Mikroorganismus, insbesondere einem E.-coli-Stamm gewonnen worden ist, der Penicillin-acylase produziert, und die der DNA-Sequenz gemäß Anspruch 1 (a) analog ist, hinter einen gegebenenfalls induzierbaren Promotor oder Promotor/Leader schaltet,
- eine DNA-Sequenz, die aus einem Mikroorganismus, insbesondere aus einem E.-coli-Stamm gewonnen worden ist, der Penicillin-acylase produziert, und die der DNA-Sequenz gemäß Anspruch 2 (d) analog ist, hinter einen gegebenenfalls induzierbaren Promotor oder Promotor/Leader schaltet und
- die beiden Untereinheiten von einem prokaryotischen Mikroorganismus bilden läßt (wobei jedes Individuum des Mikroorganismus beide Untereinheiten bildet) und gegebenenfalls extrahiert oder

- die beiden Untereinheiten für sich getrennt in verschiedenen Individuen eines prokaryotischen Mikroorganismus bilden läßt, gegebenenfalls extrahiert und gegebenenfalls danach zusammengibt und

- gegebenenfalls danach die gebildete Penicillin-acylase isoliert.

9. Verfahren nach Anspruch 8, dadurch g e k e n n - z e i c h n e t , daß man die beiden DNA-Sequenzen, die jeweils eine der beiden Untereinheiten der Penicillin-acylase kodieren, aus ein und demselben Mikroorganismus gewonnen hat.

10. Verfahren nach Anspruch 7, 8 oder 9, dadurch g e - k e n n z e i c h n e t , daß man als Promotor oder Promotor/Leader die aus ATCC 11105 gewonnenen DNA-Sequenzen gemäß Anspruch 5 einsetzt.

11. Verfahren nach einem der vorhergehenden Ansprüche, dadurch g e k e n n z e i c h n e t , daß man als prokaryotischen Mikroorganismus einen Stamm verwendet, der eine Protease-Aktivität aufweist, die der von E.-coli 5K entspricht oder größer ist, beispielsweise DSM 1454.